# EUROPEAN PATENT SPECIFICATION

(11) **EP 0 941 061 B1**
(45) Date of publication and mention of the grant of the patent: **10.07.2002**
(21) Application number: 97948899.6
(22) Date of filing: 06.11.1997
(51) Int. Cl.: A61K 7/50, A61K 7/48, A61K 7/06

(54) **ANTIMICROBIAL PERSONAL CARE COMPOSITIONS**
ANTIMIKROBIELLE KÖRPERPFLEGE-ZUSAMMENSETZUNGEN
COMPOSITIONS ANTIMICROBIENNES POUR SOINS PERSONNELS

(30) Priority: 29.11.1996 GB 9624874
(43) Date of publication of application: 15.09.1999
(73) Proprietor: UNILEVER N.V., 3013 AL Rotterdam (NL)
(72) Inventor: BHANDARY, Milind,Vinayak-A/19, Gumpha Darshan CHSH, Mumbai 400 060, Maharashtra (IN); GOUNDEN, Karuppa, Chinna-D5/504 Krishna-Kaveri CHS, Mumbai 400 053, Maharashtra (IN); KUMAR, Velayudhan, Nair,, Mumbai 400 099, Maharashtra , (IN); SINKAR, Vilas, Pandurang, Bangalore (IN); SONA, Pushker-Hindustan Lever Res. Centre Flats, Mumbai 400 099 Maharashtra (IN)
(74) Representative: Griffiths, Helen Sarah
(86) International application number: EP9706182
(87) International publication number: WO9823258

(56) References cited:
- WO-A-92/14440
- US-A- 3 723 325
- US-A- 3 769 398

## Description

### FIELD OF THE INVENTION

This invention relates to antimicrobial personal care compositions. More particularly this invention relates to personal care compositions which exhibit enhanced deposition of antidandruff agents on the surface of skin and hair.

### BACKGROUND AND PRIOR ART

WO 92/14440 relates to antidandruff shampoos comprising particulate zinc pyridinethione and a synergizer selected from a group comprising polyethylenimine.

US Patent No.4,675,178 (Calgon Corporation, USA) describes stable deodorant compositions comprising at least one antimicrobial agent and poly-dimethyldiallyl ammonium chloride-acrylamide copolymers (DMDAAC/AM), or dimethyldiallylammonium chloride cationic polymer (DMDAAC), which serve to enhance the residual efficacy of the antimicrobial agent on a surface.

US patent No.3,769,398 describes clear, viscous, homogeneous shampoo compositions which have excellent conditioning characteristics and are effective against *Pityrosporum ovale* containing an active ingredient selected from the group consisting of betaines, sulfobetaines, amine oxides, and mixtures thereof. The composition also contains a water-soluble polymer selected from the group consisting of polyethylenimine, ethoxylated polyethylenimines, and propoxylated polyethylenimines.

US patent No.3,723,325 describes detergent compositions consisting of anionic, nonionic, zwitterionic or cationic surfactants, water soluble cationic polymer of charge density greater than 0.001, and water insoluble or sparingly soluble particulate substances such as UV absorbers, ultramarine blue, silicones, and particulate antimicrobials such as heavy metal salts of 2-pyridinethiol-1-oxide (also known as pyrithione), especially the zinc and zirconium salts.

In the examples, various shampoo and shampoo formulations containing cationic polymers including polyethylenimine and its copolymer with ethylene oxide and propylene oxide, polvinylimidazole, polydimethylaminoethylmethacrylate, polydiethylaminoethylmethacrylate and quaternary amine substituted hydroxyethyl cellulose ether are disclosed.

General applications of polyethylenimine are discussed in the article "Polyethylenimine; Prospective Applications", Harold N. Feigenbaum, BASF Corporation, Clifton, New Jersey, Cosmetics & Toiletries, p.73-77, vol. 108, August 1993.

European Patent 0 093 601 B1 (Unilever PLC) describes an aqueous washing composition comprising an anionic surfactant, water-insoluble particles and a cationic polymer which serves to enhance the deposition and retention of the particles on a surface. It is disclosed that the presence of certain non-cellulosic cationic polymers of specified cationic charge density can enhance the deposition of the water insoluble particles in the absence of precipitation of cationic polymer/anionic surfactant complex. The preferred cationic polymers are those cationically substituted galactomannan gums sold by Celanese Corp. under their JAGUAR trademark series.

The prior art described above clearly indicates that the selection and performance of the cationic polymers and the conditions under which the functional ingredients show enhanced deposition of the substrates cannot be generalised and depends on a number of factors involving the nature, concentration of the polymer and other parameters, characteristic of the composition and the physical modifications achieved on the substrate.

Though several cationic polymers are known in the prior art, the selection of the particular polymer for a particular functional ingredient in certain types of formulations is unique. None of the prior art teaches the use of a cationic polymer such as polyethylenimine (PEI) in washing compositions containing piroctone olamine. The present inventors have found that the presence of PEI in washing compositions containing piroctone olamine enhances the deposition of the active piroctone olamine on to the hair or skin, leading to improved antimicrobial activity and cost effectiveness.

### SUMMARY OF THE INVENTION

The present invention provides a washing composition comprising:
a) from 2% to 50 % by weight surfactant,
b) from 0.2 % to 1.2 % by weight polyethylenimine, and
c) from 0.1 % to 3 % by wt. piroctone olamine.

A further aspect of the present invention is the use of polyethyleneimine in an amount of from 0.1% to 5% by weight to deposit piroctone olamine on to the hair or skin from a shampoo composition further comprising from 2% to 50% by weight surfactant and from 0.1 to 3% by weight piroctone olamine.

### DETAILED DESCRIPTION AND PREFERRED EMBODIMENTS

### Surfactant

The compositions in accordance with the present invention comprise one or more surfactants selected from anionic, nonionic, amphoteric, zwitterionic and cationic surfactants and mixtures thereof.

Suitable anionic surfactants are the alkyl sulphates, alkyl ether sulphates, alkaryl sulphonates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide or propylene oxide units per molecule, and preferably contain 2 to 3 ethylene oxide units per molecule.

Examples of further suitable anionic surfactants include sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

The nonionic surfactants suitable for use in the composition of the invention may include condensation products of aliphatic (C₈-C₁₈) primary or secondary linear or branched-chain alcohols or phenols with alkylene oxides, usually ethylene oxide (EO) and generally 6-30 EO groups.

Other suitable nonionics include mono- or di-alkyl alkanolamides or alkyl polyglucosides. Examples include coco mono-or di-ethanolamide, coco mono- isopropanolamide, and coco di-glucoside.

The amphoteric surfactants suitable for use in the composition of the invention may include alkyl amine oxides, alkyl betaines, alkyl amidopropyl betaines, alkyl sulpho betaines, alkyl glycinates, alkyl carboxyglycinates, alkyl amphopropionates, alkyl amidopropyl hydroxysulpho betaines, acyl laurates and acyl glutamates wherein the alkyl and acyl groups have from 8 to 18 carbon atoms. Examples include lauryl amine oxide, betaine, cocamidopropyl betaine and sodium cocamphopropionate.

Examples of cationic surfactants include: quaternary ammonium hydroxides and salts thereof, for example cetyl trimethylammonium chloride, stearyl dimethylbenzyl ammonium chloride, cetylpyridinium chloride, Quaternium-5, -31, -18 and mixtures thereof.

The level of surfactant materials in compositions of the invention is preferably 7-30% and more preferably from 12-15% by weight of the composition.

### Piroctone Olamine

Piroctone olamine has the chemical formula of C₁₅H₃₅N₂O₃ and commercially available under the brand "Octopirox".

Octopirox which is considerably substantive to scalp and hair is a known antidandruff agent. The concentration of Octopirox in a rinse-off product is from 0.1 to 3.0% by weight, preferably from 0.1 to 2.0% by weight, most preferably from 0.5 to 1% by weight.

### Polyethylenimine (PEI)

Polyethylenimine is a highly branched polyamine containing primary, secondary and tertiary amino groups in approximate proportions of 1:2:1. It exhibits high cationic charge density due to repeating C-C-N groups where the nitrogen atom can become protonated. The cationic charge gives PEI an affinity for negatively charged surfaces. The level of PEI in the composition of the invention is preferably between 0.2 to 1.2% by wt.

### Product Form

The composition according to the invention has a wide application. Preferred product forms include hair and body shampoos, hair rinse conditioners, oils and creams.

### Optional Ingredients

Compositions of this invention may contain any other ingredient normally used in washing compositions. These other ingredients may include emulsifiers, conditioning agents such as silicones, preservatives, colouring agents, chelating agents such as EDTA, antioxidants, fragrances, and sunscreens. Each of these ingredients will be present in an amount effective to accomplish its purpose. Generally these optional ingredients are included individually at a level of up to about 5% by weight of the total composition.

Preferably, shampoo compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. Arginine is a further particularly preferred amino acid nutrient.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - fatty acids, for cuticle repair and damage prevention. Examples are branched chain fatty acids such as 18-methyleicosanoic acid, and other homologues of this series, straight chain fatty acids such as stearic, myristic and palmitic acids, and unsaturated fatty acids such as oleic acid, linoleic acid, linolenic acid and arachidonic acid. Particularly preferred are those fatty acids which occur naturally as essential, integral components of the hair fibre, and which therefore may need replenishing due to fibre damage and loss. The fatty acids may be added singly, as mixtures, or in the form of blends derived from extracts of e.g. lanolin.

The invention will now be illustrated by the following nonlimiting Examples:

### EXAMPLES

Shampoo compositions according to the invention were prepared by mixing the surfactants and polyethylenimine. To this was added piroctone olamine at room temperature together with the minor and conventional ingredients.

Compositions according to the invention are shown in Examples IV to VI and Comparative Examples are shown as Examples I to III in Table 1 below:

**Table 1**

| Ingredient (% by wt.) | Ex.I | Ex.II | Ex.III | Ex.IV | Ex.V | Ex.VI |
|---|---|---|---|---|---|---|
| SLES* | 14 | 14 | 14 | 14 | 14 | 14 |
| Octopirox | - | - | 0.75 | 0.75 | 0.75 | 0.75 |
| PEI | - | 0.6 | - | 0.2 | 0.6 | 1.2 |
| Perfume | 0.8 | 0.8 | 0.8 | 0.8 | 0.8 | 0.8 |
| Convent'l# ingredients | 3.8 | 3.8 | 3.8 | 3.8 | 3.8 | 3.8 |
| Water | to 100% | to 100% | to 100% | to 100% | to 100% | to 100% |

| | | | | | | |
|---|---|---|---|---|---|---|
| * Sodium lauryl ether sulphate. | | | | | | |
| # Conventional conditioning and suspending polymers and thickening agents. | | | | | | |

### Deposition of Octopirox to hair:

8.33 g of each of the formulations given in Examples I to IV was diluted to 25 ml. using distilled water. 2g of prewashed human hair swatches were immersed in the above solutions for 5 minutes and agitated gently. A corresponding blank in distilled water was maintained to obtain a background reading. After 5 minutes the swatches were removed from the shampoo solutions and the adhering liquid was drained off. It was rinsed with 200 ml of water twice. The hair was then washed with 10 ml of alcohol twice and the washings were pooled and volume was made up to 25 ml. The UV absorbance of the above solution was measured at 305 nm and the % Octopirox was estimated as indicated in table 2. Octopirox has UV absorption with a peak at 305 nm. This peak was used to quantify the amount of residual Octopirox extracted from hair/skin. However, merely treating skin/hair with shampoo in the presence and absence of PEI gives rise to residual binding of shampoo ingredient to substrate which gets extracted in methanol. This extract showed a UV absorbance which flattened near 305nm. Therefore in estimating the amount of Octopirox, the absorbance at 305 nm (absorption maxima for Octopirox) for samples containing Octopirox with or without PEI is subtracted from base absorbance observed in the control (Example III). The data is quantified with the aid of a calibration curve.

Data presented in Table 2 shows that the presence of PEI in the formulation significantly increases the binding of Octopirox to hair. The formulation in Example V (where 0.6% PEI was present in the shampoo) showed an Octopirox binding of 0.736 mg of Octopirox/ 2 gram hair as compared to 0.471 mg/2 gram hair in the case of Example III which contained 0% PEI.

**Table 2**

| Effect of PEI on Octopirox binding to hair from shampoo | | | | |
|---|---|---|---|---|
| Formulation | A Absorbance at 305nm Control | B Absorbance at 305nm SAMPLE | A-B Residual absorbance due to Octopirox | mg Octopirox bound on 2 g of hair |
| Example I 0% Octopirox 0% PEI | 0.142 (A_{I}) | - | - | - |
| Example II 0% Octopirox 0.6% PEI | 0.337 (A_{II}) | - | - | - |
| Example III 0.75% Octopirox 0% PEI | - | 0.604 (A_{III}) | 0.462 (A_{III} - A_{I}) | 0.471 |
| Example IV 0.75% Octopirox 0.2% PEI | - | 0.663 (A_{IV}) | 0.326 (A_{IV} - A_{II}) | 0.332 |
| Example V 0.75% Octopirox 0.6% PEI | - | 1.058 (A_{V}) | 0.721 (A_{V} - A_{II}) | 0.736 |
| Example VI 0.75% Octopirox 1.2% PEI | - | | 0.32 (A_{VI} - A_{II}) | 0.326 |

### Deposition of Octopirox to skin:

8.33 g of each of the formulations given in Examples I to VI were diluted to 25 ml. using distilled water. 0.5 ml of these solutions were spread on guinea pig skin (~5 cm²) and left for 5 minutes. An untreated blank was also maintained to obtain a background reading. The skin was then dabbed and rinsed with 50 ml water twice.

The skin was washed with 5 ml alcohol three times and the washings were pooled and volume made up to 25 ml. The UV absorbance of the above solution was measured at 305 nm and the % Octopirox was estimated as indicated in table 3. The data is quantified with the aid of a calibration curve.

The data presented in Table 3 shows that there is a significant enhancement in binding of Octopirox in the presence of PEI. Increased Octopirox binding on skin was shown by a formulation containing 0.6% PEI (Example V) as compared to one containing 0% PEI (Example III).

**Table 3**

| Effect of PEI on Octopirox binding on skin from shampoo | | | | |
|---|---|---|---|---|
| Formulation | A Absorbance at 305nm CONTROL | B Absorbance at 305nm SAMPLE | A-B Residual absorbance due to Octopirox | mg Octopirox bound on 5 sq.cm. of skin |
| Example I 0% Octopirox 0% PEI | 0.056 (A_{I}) | - | - | - |
| Example II 0% Octopirox 0.6% PEI | 0.078 (A_{II}) | - | - | - |
| Example III 0.75% Octopirox 0% PEI | - | 0.176 (A_{III}) | 0.12 (A_{III} - A_{I}) | 0.122 |
| Example IV 0.75% Octopirox 0.2% PEI | - | 0.199 (A_{IV}) | 0.121 (A_{IV} - A_{II}) | 0.123 |
| Example V 0.75% Octopirox 0.6% PEI | - | 0.217 (A_{V}) | 0.139 (A_{V} - A_{II}) | 0.141 |
| Example VI 0.75% Octopirox 1.2 PEI | - | 0.075 (A_{VI}) | - (A_{VI} - A_{II}) | - |

## Claims

1. A washing composition comprising:
a) from 2% to 50% by weight surfactant,
b) from 0.2% to 1.2% by weight polyethylenimine, and
c) from 0.1% to 3% by weight piroctone olamine.

2. A washing composition according to Claim 1 which is a hair shampoo and in which the surfactant is selected from the group consisting of anionic surfactants, amphoteric surfactants and mixtures thereof.

3. A washing composition according to Claim 1 or Claim 2 in which the level of surfactant is from 7 to 30% by weight of the composition.

4. A washing composition according to any one of Claims 1 to 3 in which the level of piroctone olamine is from 0.5 to 1% by weight.

5. A washing composition according to any one of Claims 1 to 4 which further comprises a silicone.

6. The use of polyethylenimine in an amount of from 0.1% to 5% by weight to deposit piroctone olamine on to the hair or skin from a shampoo composition further comprising from 2% to 50% by weight surfactant and from 0.1% to 3% by weight piroctone olamine.

## Patentansprüche

1. Waschzusammensetzung, umfassend:
a) 2 % bis 50 Gew.-% Tensid
b) 0,2 % bis 1,2 Gew.-% Polyethylenimin und
c) 0,1 % bis 3 Gew.-% Pirocton-Olamin.

2. Waschzusammensetzung nach Anspruch 1, die ein Haarshampoo darstellt und worin das Tensid aus der Gruppe, bestehend aus anionischen Tensiden, amphoteren Tensiden und Gemischen davon, ausgewählt ist.

3. Waschzusammensetzung nach Anspruch 1 oder Anspruch 2, wobei der Anteil an Tensid 7 bis 30 Gew.-% der Zusammensetzung ausmacht.

4. Waschzusammensetzung nach einem der Ansprüche 1 bis 3, wobei der Anteil an Pirocton-Olamin 0,5 bis 1 Gew.-% ist.

5. Waschzusammensetzung nach einem der Ansprüche 1 bis 4, die weiterhin ein Silicon umfasst.

6. Verwendung von Polyethylenimin in einer Menge von 0,1 % bis 5 Gew.-% zum Abscheiden von Pirocton-Olamin auf das Haar oder die Haut aus einer Shampoozusammensetzung, die weiterhin 2 % bis 50 Gew.-% Tensid und 0,1 % bis 3 Gew.-% Pirocton-Olamin umfasst.

## Revendications

1. Composition nettoyante comprenant:
a) de 2 % à 50 % en poids de tensioactif ;
b) de 0,2 % à 1,2 % en poids de polyéthylènenimine ; et
c) de 0,1 % à 3 % en poids de piroctone olamine.

2. Composition nettoyante selon la Revendication 1, qui est un shampoing pour les cheveux et dans laquelle le tensioactif est sélectionné à partir du groupe composé des tensioactifs anioniques, des tensioactifs amphotères et des mélanges de ceux-ci.

3. Composition nettoyante selon la Revendication 1 ou la Revendication 2, dans laquelle le niveau de tensioactif est de 7 à 30 % en poids de la composition.

4. Composition nettoyante selon l'une quelconque des Revendications 1 à 3, dans laquelle le niveau de piroctone olamine est de 0,5 à 1 % en poids.

5. Composition nettoyante selon l'une quelconque des revendications 1 à 4, comprenant en outre une silicone.

6. Utilisation de polyéthylèneimine dans une quantité allant de 0,1 % à 5 % en poids afin de déposer du piroctone olamine sur les cheveux ou sur la peau à partir d'une composition de shampoing comprenant en outre de 2 % à 50 % en poids de tensioactif et de 0,1 % à 3 % en poids de piroctone olamine.
